# EUROPÄISCHE PATENTSCHRIFT

(11) **EP 2 022 541 B1**
(45) Veröffentlichungstag und Bekanntmachung des Hinweises auf die Patenterteilung: **18.06.2014**
(21) Anmeldenummer: 08014204.5
(22) Anmeldetag: 08.08.2008
(51) Int. Cl.: A63B 71/06, A63B 21/00, A63B 23/18, A61B 5/00

(54) **Tragbarer Atemtakter**
Portable breath meter
Appareil respiratoire portatif

(30) Priorität: 08.08.2007 DE 102007037408
(43) Veröffentlichungstag der Anmeldung: 11.02.2009
(73) Patentinhaber: Fehrl, Heidrun, 38116 Braunschweig (DE)
(72) Erfinder: Fehrl, Heidrun, 38116 Braunschweig (DE)
(74) Vertreter: Lins, Edgar

(56) Entgegenhaltungen:
- EP-A- 0 856 334
- WO-A-94/14374
- FR-A- 2 599 629
- US-A1- 2004 224 822
- US-B1- 7 199 700

## Beschreibung

Die Erfindung betrifft einen Atemtakter, mit einer Signaleinheit (5, 6) zum wahrnehmbaren Übertragen eines Signals auf den Körper des Benutzers und mit einer Steuereinheit zum periodischen Ansteuern der Signaleinheit (5, 6) mittels eines Ansteuersignals, wobei eine Periode des Ansteuersignals eine Einatmungsphase und eine Ausatmungsphase umfasst, wobei das Ansteuersignal unabhängig von physiologischen Körperwerten gebildet ist, das Signal ein Vibrationssignal, ein Stromimpulssignal und/oder ein Temperatursignal ist, und die Dauer der Einatmungsphase nicht länger als die Dauer der Ausatmungsphase ist, und dass mit mehreren Perioden eine voreingestellte Sequenz einer Atemübung gebildet ist und dass die zeitliche Abstände der einzelnen sequenzen untereinander einstellbar sind.

Ein Atemtakter dieser soll einen Benutzer in die Lage versetzen, eine gesunde Atmung zu erwerben, in akuten Stresssituationen seine Körperfunktionen (Puls, Herzschlag, vegetatives Nervensystem) zu kontrollieren und die Stressbelastung durch Cortisol etc. sofort zu reduzieren.

Ein Atemtakter gibt ein von einem Benutzer wahrnehmbares Signal aus, das ihm die Einatmungsphasen, Ausatmungsphasen und ggf. Pausenphasen vorgibt. An diesen Phasen richtet der Benutzer seine Atmung aus.

Eine gesunde Atmung ist wichtig für eine Vorbeugung von Stresserkrankungen. Physische und psychische Stresserkrankungen nehmen insbesondere in Industriegesellschaften stetig zu. Stress ist Auslöser zahlreicher gesundheitlicher Probleme mit den daraus resultierenden betriebswirtschaftlichen und gesamtgesellschaftlichen Kosten.

Stress kann für viele Folgeerkrankungen verantwortlich sein, beispielsweise für Bluthochdruck, Rückenschmerzen, Herz-Kreislauf-Erkrankungen, Angina Pectoris, Herzinfarkt und Schlaganfall. Darüber hinaus verursacht Stress Konzentrations- und Leistungsstörungen, chronische Erschöpfung, Angsterkrankungen, Panikattacken und Depressionen. Zudem steht Stress im Verdacht, die Gefahr von Tumorerkrankungen zu erhöhen. Stress kann auch chronisch werden und die Gesundheit dauerhaft beeinträchtigen und gefährden.

Aus der DE 693 19 742 T2 ist ein Atemregler bekannt, mit dem Atmungsregelmäßigkeitsstörungen behandelt werden. Der Atemregler bestimmt das Verhältnis zwischen der Einatmungszeit und der Ausatmungszeit in einem Atmungszyklus. In Abhängigkeit dieses Verhältnisses wird ein von dem Benutzer wahrnehmbares akustisches Signal ausgegeben, an dem der Benutzer seine Atmung ausrichtet. Wenn der Atemregler durch Auswertung des Verhältnisses feststellt, dass die Atmung des Benutzers wieder mit einem vorgegebenen gesunden Atmungsmuster übereinstimmt, schaltet er sich selbsttätig ab. Nachteilig ist, dass der Atemregler einen komplizierten technischen Aufbau aufweist und damit kostspielig in der Herstellung ist. Zudem ist er nicht einfach zu bedienen. Ein weiterer Nachteil besteht darin, dass er auffällig und für Dritte wahrnehmbar ist.

Ein Atemtakter der eingangs erwähnten Art ist durch US 2004/224822 A1 bekannt. Das Gerät kann beispielsweise die Form einer Armbanduhr aufweisen und kann für eine Atemperiode programmiert werden, in der die Dauer des Einatmens, eine Pausedauer, die Dauer des Ausatmens und eine weitere Pausendauer eingestellt werden. Dementsprechend gibt das Gerät Signale ab, nach denen der Benutzer erkennen kann, wie lange er Einatmen soll, wie lange eine Pausendauer sein soll und wie lange er ausatmen soll. Die Signalgabe kann beispielsweise durch Anzeigen an dem Gerät erfolgen. Eine spürbare Anzeige kann dadurch erfolgen, dass Taststifte von dem Gerät in Richtung Haut des Benutzers ausgefahren werden, sodass der Benutzer durch das Verspüren des Drucks durch die Taststifte eine Signalisierung erfährt, Durch die Einstellbarkeit des Geräts sind unterschiedliche Perioden für einen Atemzyklus mit unterschiedlichen Einatmungs- und Ausatmungszeiten einstellbar,

Aus der US 7,199,700 B1 geht ein Atmungserinnerungsgerät hervor, das in gewissen Zeitabständen, z. B. 15 Minuten, über eine Sequenz von Vibrationssignalen einen Benutzer daran erinnert, eine gesunde Atmung auszuführen.

Aus der EP 0856334 A2 ist ein therapeutisches Gerät mit einer visuellen Stimulierungseinrichtung bekannt, die einem Benutzer dabei behilflich ist, seine Atemfrequenz zu verringern. Hiefür ist bei dem dortigen Gerät vorgesehen, zyklische grafische Darstellungen auf einem Display anzuzeigen, das aus einem Raster von Leuchtdioden besteht.

Aufgabe der vorliegende Erfindung ist es, ein einfach herzustellendes, kostengünstiges und auch von Kindern - z. B. mit ADHS (Hyperaktivität) - leicht bedienbares Gerät bereitzustellen, mit dem ein Benutzer eine gesunde Atmung erlernen kann und dass die Atmung des Benutzers auf Dauer positiv beeinflussen kann.

Diese Aufgabe wird mit einem Atemtakter der eingangs erwähnten Art dadurch gelöst, dass mit mehreren Perioden eine voreingestellte Sequenz einer Atemübung gebildet ist und dass die zeitlichen Abstände der einzelnen Sequenzen untereinander einstellbar sind.

Die vorliegende Erfindung beruht auf der Erkenntnis, dass das Ergebnis der Atemübungen davon abhängt, dass die Atemübungen regelmäßig und mit angemessener täglicher Dauer durchgeführt werden. Demzufolge stellt der erfindungsgemäße Atemtakter voreingestellte zeitliche Sequenzen der Benutzung zur Verfügung, beispielsweise Sequenzen von fünf Minuten bis zu einer Stunde. Die Abstände der einzelnen Sequenzen untereinander sind einstellbar. Beispielsweise kann eine Sequenz mit sechs Atemzügen pro Minute für eine Dauer von 10 Minuten eine Atemübung definieren. Der Abstand zwischen einzelnen Atemübungen ist einstellbar und kann beispielsweise 90 Minuten betragen.

Der erfindungsgemäße Atemtakter umfasst einfache und kostengünstige Komponenten, wie zum Beispiel eine Signaleinheit zum Übertragen einer Vibration, eines Stromimpulses oder einer Temperaturänderung. Diese Signale können einzeln oder in beliebiger Kombination übertragbar sein. In einer einfachen Ausführungsform erfolgen die Signale gleichmäßig, also beispielsweise mit einer gleichmäßig starken Vibration beim Einatmen undloder Ausatmen.

Die Steuereinheit generiert ein periodisches Ansteuersignal, mit dem die Signaleinheit angesteuert wird und ein entsprechendes periodisches Signal ausgibt, das von einem Benutzer wahrnehmbar ist. Die Steuereinheit kann hierzu einen geeignet programmierten Mikrocontroller umfassen. Die Daten für das Ansteuersignal entnimmt die Steuereinheit beispielsweise einem geeigneten Speicher, der in dem Atemtakter integriert ist.

Die unterschiedlichen Phasen (Einatmungs-, Ausatmungs- und ggf. Pausenphase) sind von dem Benutzer unterschiedlich wahrnehmbar, damit er sie auseinander halten kann, Dies ist wichtig, da er seine Atmung an diesen Phasen ausrichten muss, um den gewünschten Zweck, eine gesündere Atmung, zu erreichen. Die Erfindung umfasst auch den Fall, dass eine Phase dadurch gekennzeichnet ist, dass kein positiv wahrnehmbares Signal abgegeben wird, wenn die zeitlich unmittelbar angrenzenden Phasen positive Signale umfassen. Entscheidend ist die Erkennbarkeit der einzelnen Phasen für den Benutzer. Positiv wahrnehmbare Signale können gleichmäßig oder ungleichmäßig sein.

Der erfindungsgemäße Atemtakter verzichtet im Gegensatz zu vielen bekannten Atemregler auf eine Regeleinrichtung, die mit komplizierten elektronischen Schaltungen realisiert ist und geeignete Sensoren zum Detektieren von physischen Zuständen des Benutzers benötigt. Eine Regeleinrichtung, einschließlich der erforderlichen Sensoren, ist kostspielig. Zudem macht sie die Bedienung des Atemreglers kompliziert, was Kinder und ältere Menschen sowie Menschen mit geringen intellektuellen Fähigkeiten in der Nutzung einschränken würde.

Der erfindungsgemäße Atemtakter ist jedoch in der Lage, die Atmung des Benutzers auf Dauer genauso positiv zu beeinflussen, wie die komplizierten bekannten Geräte. Es hat sich nämlich gezeigt, dass das Ergebnis der Atemübung davon abhängt, dass die Atemübung regelmäßig und von angemessener täglicher Dauer durchgeführt wird.

Der erfindungsgemäße Atemtakter ist tragbar und erfordert keine visuelle Kontrolle. Der Benutzer braucht also nicht zu liegen oder seine visuelle Aufmerksamkeit dem Atemtakter zu widmen. Es kann deshalb nahezu überall eingesetzt werden, beispielsweise beim Autofahren, beim Halten von Vorträgen, in Gefahrensituationen (Feuerwehr, Rettungspersonal, Taucher etc.) oder generell in Situationen, die Konzentration und Aufmerksamkeit erfordern.

Der erfindungsgemäße Atemtakter wirkt sich positiv auf die Gesundheit des Benutzers aus, insbesondere durch eine Erhöhung der Herzratenvariabilität (HRV: Schwankungen der Herzfrequenz von Schlag zu Schlag) und Herzkohärenz (Zustand der Synchronisation von Atmung, Herzschlag und Blutdruck) sowie durch ein aktives Entgegenarbeiten einer schädlichen Hyperventilation bei Menschen mit Hyperventilationssyndrom. Der HRV-Wert ist ein Parameter, der physiologische Zustände anzeigt. Mit ihm werden Stressbelastungen und Zustände des Organsystems gemessen. Der gemessene HRV-Wert ist ein zuverlässiger Indikator für unterschiedliche Störungen. Die Erhöhung der Anpassungsleistung des Herzens (Herzkohärenz) durch Einsatz des Atemtakters führt zu Prävention und Rückgang von riskanten Organzuständen. Zu nennen sind etwa:
■ Stressbedingt hoher Blutdruck wird ohne Medikamente gesenkt
■ Ein- und Durchschlafstörungen werden verringert
■ Ängste und Panikstörungen können besser kontrolliert werden
■ Herz- und Kreislauf werden wieder ins Gleichgewicht gebracht (bei erlittenem Herzinfarkt besteht bei einer hohen HRV eine neunmal bessere Chance, keinen zweiten Herzinfarkt zu erleiden)
■ Bei kurz dauernder Diabetes wird die Zerstörung des Vagusnervs hinausgezögert
■ Die Produktion schädlicher Stresshormone wird gedrosselt
■ Die DHEA-Werte (DHEA; Dehydroepiandrosteron, Zellerneuerungshormon) erhöhen sich bei einer täglichen Atemübung von insgesamt dreißig Minuten nach achtundzwanzig Tagen bereits um 100%
■ Die Immunkräfte werden gestärkt
■ Konzentration und Leistungsfähigkeit werden gesichert
■ Die generelle Stressresistenz wird erhöht
■ Zusammen mit Stretching verdoppelt sich die HRV in nur achtundzwanzig Tagen (selbst bei seit einem Jahr bestehendem Muskel- und Kreislauftraining)
■ Die Alterung wichtiger Organsysteme wird durch eine hohe HRV gehemmt

Für die Stromversorgung des erfindungsgemäßen Atemtakters wird vorzugsweise eine Batterie verwendet, die in dem Atemtakter austauschbar integriert ist. Geeignete Batterien sind beispielsweise von Armbanduhren bekannt. Der Atemtakter kann jedoch auch mit einem Akkumulator versehen sein, der über ein anschließbares Ladekabel oder eine Solarzelle aufladbar ist. Es ist auch möglich, dass der Akkumulator über einen automatischen Aufzug aufgeladen wird. Hierzu weist der Atemtakter beispielsweise ein bewegliches Teil auf (z.B. eine in einer Führungsnut umlaufende Kugel), das über Bewegungen des Atemtakters in Bewegungen versetzt wird und über einen kleinen Generator den Akkumulator auflädt, indem dessen kinetische Energie in elektrische Energie umgewandelt wird.

Vorteilhaft ist es, wenn der Atemtakter ein Befestigungsmittel aufweist, um ihn am Körper des Benutzers zu befestigen. Geeignete Befestigungsmittel sind beispielsweise ein Brustband, Bauchgurt, Armband, Headset, eine anatomisch oder nichtanatomisch geformte Ohrklammer, eine Klettvorrichtung, Heftklemme, Heftklammer, Öse oder eine Ohrkapsel. Es ist jedoch auch möglich, dass der Atemtakter lose in einer Tasche getragen wird oder auf einem "Klangkörper" (beispielsweise einem Tisch) liegt, der das Vibrationssignal akustisch hörbar macht. Dabei ist darauf zu achten, dass die Signale vom Benutzer wahrgenommen werden können. Der Atemtakter lässt sich auch in anderes Gerät integrieren, beispielsweise in ein Handy. Der Atemtakter kann auch in Kleidungsstücke, Autositze oder dergleichen, mit denen der Benutzer bestimmungsgemäß in Körperkontakt kommt, integriert werden. Das Befestigungsmittel kann von dem Atemtakter abnehmbar ausgebildet sein.

In einer Ausführungsform ist erfindungsgemäß vorgesehen, dass das Signal zusätzlich ein mechanisches, akustisches und/oder optisches Signal umfasst. Durch das zusätzliche akustische und/oder optische Signal wird die Wahrnehmbarkeit des Signals verbessert oder für mehrere Personen gleichzeitig sichtbar gemacht. Als akustisches Signal kommt im Prinzip jeder Ton in Betracht, bevorzugt ein Ton eines Instruments, eine Stimme oder ein natürliches Geräusch.

Vorteilhaft ist es, wenn das Signal der Signaleinheit einstellbar ist. Beispielsweise lässt sich die Lautstärke des akustischen Signals durch einen an dem Atemtakter vorgesehenen Lautstärkeregler verändern.

Zweckmäßig ist es, wenn die Periodendauer, die Dauer der Einatmungsphase, die Dauer der Ausatmungsphase und die Dauer einer optionalen Pausenphase einstellbar sind. Dies ermöglicht eine Anpassung an verschiedene Benutzer mit unterschiedlichen Atmungsrhythmen, beispielsweise für die verschiedenen Personen einer Familie. So können mehrere Personen ein einziges Gerät benutzen. Dies erhöht die Wirtschaftlichkeit des erfindungsgemäßen Atemtakters. Zweckmäßig weist der Atemtakter einen Speicher mit Speicherplätzen für individuelle Einstellungen des Atemtakters (z. B. Abstände der Atemübungen, Vibrationsstärke, Lautstärke, Temperatur) auf.
Die einzelnen Einstellungen lassen sich über ein geeignetes Eingabeelement (z. B. einen Knopf) abrufen.

Erfindungsgemäß ist auch vorgesehen, dass die Dauer einer Atemübung einstellbar ist. Nach Ablauf der Dauer schaltet sich der Atemtakter selbständig aus. Auf diese Weise wird eine sogenannte "Schlummerfunktion" bereitgestellt.

Es hat sich als vorteilhaft herausgestellt, wenn die Dauer der Einatmungsphase nicht länger ist als die Dauer der Ausatmungsphase, insbesondere wenn die Einatmungsphase kürzer - vorzugsweise etwa 1 bis 2 Sekunden kürzer - ist als die Dauer der Ausatmungsphase bzw. wenn pro Minute bevorzugt 5 bis 10 (je nach Gewicht und Bedarf des Anwenders) komplette Atemzyklen stattfinden, die das physiologische Gleichgewicht optimal regulieren, wie aus neuesten Forschungsergebnissen hervorgeht. Aus diesem Grunde ist die erfindungsgemäße Atemtakter entsprechend ausgebildet. In einer besonderen Ausführungsform weist die Einatmungsphase eine Dauer von 1 bis 10 und die Ausatmungsphase eine Dauer von 2 bis 20. Optional kann nach der Ausatmungsphase eine Pausenphase vorgesehen sein. Diese hat zweckmäßig eine Dauer von 0,5 bis 2 Sekunden, vorzugsweise von etwa 1 Sekunde.

Erfindungsgemäß ist vorgesehen, dass die Signaleinheit einen Lautsprecher, eine Leuchtdiode, ein Temperaturelement, ein Stromimpulselement undloder ein Vibrationselement aufweist. Mit diesen Elementen können die Signale auf einfache Art und Weise für den Benutzer wahrnehmbar übertragen werden. Der Lautsprecher ist vorzugsweise am Ohr vorgesehen, beispielsweise an einer Ohrklammer. Die Leuchtdiode ist vorzugsweise zum Tragen dicht an den Augen vorgesehen, beispielsweise an einer Brille bzw. einem Brillengestell. Das Temperaturelement ist vorzugsweise als isoliertes oder unisoliertes Metallelement, beispielsweise ein Metallstreifen, zum Tragen auf der Haut vorgesehen. Das Vibrationselement ist vorzugsweise als Exzenter zum Tragen auf der Haut vorgesehen. Es ist jedoch auch möglich, das Vibrationselement als Lautsprecher mit einer Lautsprechermembran auszubilden, die in Schwingungen mit niedrigen Frequenzen versetzt wird. Diese niederfrequenten Schwingungen sind als Vibration haptisch wahrnehmbar. Das Vibrationselement kann auch in der Hosentasche etc. getragen werden, wenn die Vibration ausreichend stark ist bzw. sich ausreichend stark einstellen lässt.

In einer bevorzugten Ausführungsform ist vorgesehen, dass das Signal während der Einatmungsphase eine Vibration zunehmender Intensität und/oder zunehmender Frequenz aufweist, wobei die Zunahme vorzugsweise gleichmäßig erfolgt. Zweckmäßig ist es in diesem Fall (aber nicht nur in diesem Fall), wenn das Signal während der Ausatmungsphase eine Vibration abnehmender Intensität und/oder abnehmender Frequenz aufweist, wobei die Abnahme vorzugsweise gleichmäßig erfolgt. Es hat sich nämlich gezeigt, dass die Menschen ihre Atmung gerne an Signalen ausrichten, die sich gleichmäßig verändern. Selbstverständlich kann die Vibration während der Ausatmungsphase auch eine zunehmende Intensität und/oder Frequenz aufweisen.

Denkbar ist es aber auch, dass das Signal während der Einatmungsphase eine Vibration abnehmender Intensität und/oder abnehmender Frequenz aufweist, wobei die Abnahme vorzugsweise gleichmäßig erfolgt. Zweckmäßig ist es in diesem Fall (aber nicht nur in diesem Fall), wenn das Signal während der Ausatmungsphase eine Vibration zunehmender Intensität und/oder zunehmender Frequenz aufweist, wobei die Zunahme vorzugsweise gleichmäßig erfolgt.

Ist das Signal ein Stromimpuls bzw. eine Stromimpulsfolge, so kann es in einer Phase mit positivem Signal gleichmäßig, zunehmend oder abnehmend gebildet sein.

Eine weitere Ausführungsform mit sich verändernden Signalen erhält man, wenn das Signal während der Einatmungsphase eine sich räumlich fortpflanzende oder ausbreitende Vibration und während der Ausatmungsphase eine sich entgegengesetzt zu der Einatmungsphase verhaltende Vibration aufweist. Zweckmäßig erfolgt die Veränderung bei dieser Ausführungsform gleichmäßig. Mit räumlich fortlaufend ist gemeint, dass die Fläche der Vibration konstant bleibt, sich jedoch der Ort (etwa auf der Haut) der Vibration ändert, Bei der sich ausbreitenden Vibration ändert sich nicht nur der Ort (ein neuer Ort kommt hinzu), sondern auch die Fläche der Vibration, beispielsweise eine sich kreisflächenförmig ausbreitende Vibration. In der Ausatmungsphase zieht sich die Kreisfläche der Vibration bei der vorstehend genannten Ausführungsform wieder zusammen (kleiner werdende Kreise).

Mit einem Temperaturelement lässt sich ein veränderndes Signal realisieren, wenn das Signal während der Einatmungsphase eine zunehmende Temperatur und während der Ausatmungsphase eine abnehmende Temperatur aufweist oder umgekehrt. Vorzugsweise erfolgt die Veränderung der Temperatur gleichmäßig.

Es hat sich herausgestellt, dass gute gesundheitliche Fortschritte erzielt werden, wenn die Atmung regelmäßig trainiert wird. Aus diesem Grunde ist erfindungsgemäß vorgesehen, dass die Steuereinheit ausgebildet ist, zu überwachen, ob eine vorgegebene Benutzungsdauer des Atemtakters in einer vorgegebenen Zeitspanne erreicht wird. Beispielsweise beträgt die vorgegebene Benutzungsdauer dreißig Minuten und die vorgegebene Zeitspanne vierundzwanzig Stunden. Zweckmäßig wird noch ein Startzeitpunkt festgelegt, beispielsweise eine bestimmte Uhrzeit. Hat der Benutzer seine Benutzungsdauer für die Zeitspanne noch nicht erfüllt, kann vorgesehen sein, dass der Atemtakter eine Warnung, beispielsweise als akustischen Ton oder optisches Signal, ausgibt. Vorzugsweise erfolgt die Warnung so rechtzeitig, dass die Benutzungsdauer noch erreicht werden kann, Die Warnung kann auch mehrmals und mit zunehmender Intensität erfolgen.

Der Atemtakter kann auch ohne zeitliche Begrenzung aktiviert werden. Dies ist in akuten Stresssituationen, bei Schlaflosigkeit etc. sinnvoll.

In einer besonderen Ausführungsform ist der erfindungsgemäße Atemtakter wasserdicht. So kann es auch draußen bei Regen oder im Wasser benutzt werden. Für die Benutzung des erfindungsgemäßen Atemtakters durch Taucher ist wichtig, dass der Atemtakter ausreichend stabil ist, um den auftretenden Drücken im Wasser standzuhalten. Als Grundregel gilt, dass der Druck um etwa 1 bar je 10 m Wassertiefe zunimmt. So muss der Atemtakter für Tauchgänge bis zu 20 m Wassertiefe einen Druck von mindestens 3 bar standhalten, bei 30 m Wassertiefe bereits 4 bar. Bei den für den Atemtakter verwendeten Materialien, beispielsweise für ein Gehäuse oder eine Dichtung, ist zweckmäßig zu berücksichtigen, ob der Atemtakter in Süßwasser- oder Salzwasser-Gewässern eingesetzt werden soll.

Erfindungsgemäß ist weiter eine Messeinrichtung zum Messen einer oder mehrerer Körperfunktionen vorgesehen. Zweckmäßig weist der Atemtakter eine Warneinrichtung zum Warnen des Benutzers auf, wenn mindestens ein Grenzwert einer gemessenen Körperfunktion erreicht oder überschritten wird.

Der Benutzer kann seine Übungen verfolgen, wenn Informationen zu durchgeführten Übungseinheiten in einem Speicher abgespeichert auf einer Anzeige angezeigt werden können.

Erfingdungsgemäß ist ferner eine "Pausefunktion" vorgesehen. Hierzu ist der Atemtakter eingerichtet, eine laufende Übung zu unterbrechen, um sie später fortzusetzen. Die "Pausefunktion" lässt sich zweckmäßig durch Drücken eines entsprechenden Knopfes auslösen und/oder beenden. Denkbar ist auch, dass sich der Atemtakter nach einer vorgegebenen Zeitdauer automatisch wieder einschaltet (sogenannte "Weckfunktion").

Die Erfindung wird anhand des in der folgenden Figur dargestellten Ausführungsbeispiels näher erläutert. Es zeigt
- Figur 1: eine Ausführungsform des erfindungsgemäßen Atemtakters,
- Figur 2: eine perspektivische Draufsicht auf eine weitere Ausführungsform des erfindungsgemäßen Atemtakters und
- Figur 3: eine perspektivische Rückansicht des Atemtakters aus Figur 2.

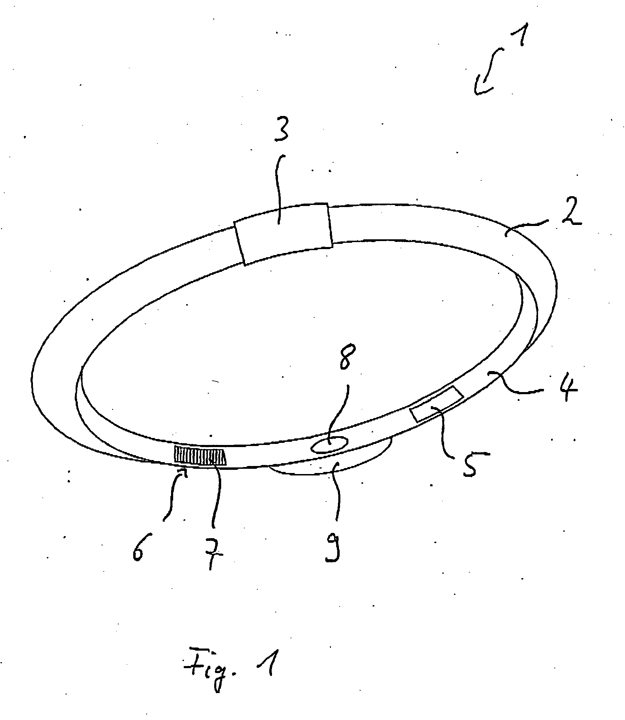

In Figur 1 ist ein tragbarer Atemtakter 1 dargestellt.

Der Atemtakter 1 umfasst ein Armband 2. Das Armband 2 lässt sich an einem Verschluss 3 öffnen und schließen.

An der Innenseite 4 (der einem Arm zugewandten Seite) des Armbandes 2 ist ein Vibrationselement 5 angebracht. Das Vibrationselement 5 wird von einer nicht dargestellten Steuereinheit mit einer Folge von Ansteuersignalen derart angesteuert, dass eine Folge von Einatmungs-, Ausatmungs- und Pausenphasen gebildet wird. Die Einatmungsphase hat eine Dauer von vier Sekunden, die Ausatmungsphase von sechs Sekunden. Eine Pausenphase ist in diesem Beispiel nicht vorgesehen.

Die Steuereinheit wird über einen nicht dargestellten Ein/Ausschalter ein- bzw. ausgeschaltet. Der Ein/Ausschalter ist an dem Armband 2 angeordnet.

An der Innenseite 4 ist ein Temperaturelement 6 angebracht. Das Temperaturelement 6 weist eine Mehrzahl im Wesentlichen paralleler Drähte 7 auf. Das Temperaturelement 6 ist mit der Steuereinheit verbunden. Die vorstehende Folge von Einatmungs-, Ausatmungs- und Pausenphasen wird in Form von unterschiedlichen Temperaturen des Temperaturelements 6 gebildet. Die Drähte sind aus Kupfer hergestellt, um eine ausreichend schnelle Temperaturänderung zu ermöglichen. Weitere geeignete Materialien für die Drähte sind Silber und Gold.

An der Innenseite 4 ist ein Pulsmesser 8 angebracht, der den Puls des Benutzers misst. Über eine LED 9 (Light Emission Diode) wird der Pulsschlag optisch angezeigt. Der gemessene Puls hat jedoch keinen Einfluss auf die Signalfolge. Es ist möglich, weitere Sensoren für physiologische Körperwerte vorzusehen, etwa für den Blutdruck oder das Erreichen der optimalen HRV bzw. Herzkohärenz, die ebenfalls keinen Einfluss auf die Signalfolge haben.

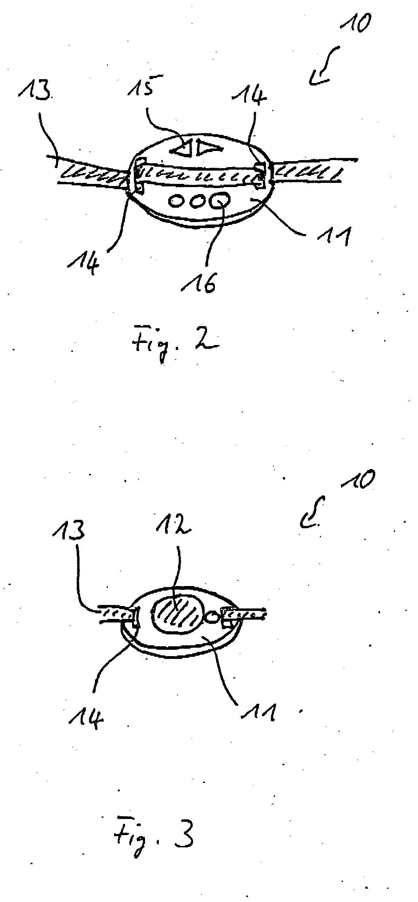

Figur 2 zeigt eine weitere Ausführungsform eines erfindungsgemäßen Atemtakters 10.

Der Atemtakter 10 umfasst ein Gehäuse 11, in das eine Signaleinheit 12 und eine nicht dargestellte Steuereinheit integriert sind.

Der Atemtakter 10 umfasst ferner ein Brustband 13 als Befestigungsmittel.

Das Brustband 13 ist durch zwei Langlöcher 14 durchgezogen, die an dem Gehäuse 11 ausgebildet sind. Durch diese Verbindung des Gehäuses 11 und des Brustbandes 13 ist es möglich, das Gehäuse 11 relativ zum Brustband 13 zu verschieben.

Der Atemtakter 10 weist Bedienelemente 15, 16 auf.

Die Bedienelemente 15 dienen der Einstellung der Stärke des Signals der Signaleinheit 12.

Mit den Bedienelementen 16 lassen sich weitere Einstellungen vornehmen, beispielsweise die Auswahl von Personeneinstellungen oder Signaleinstellungen.

### Bezugszeichenliste

- 1: Atemtakter
- 2: Armband
- 3: Verschluss
- 4: Innenseite
- 5: Vibrationselement
- 6: Temperaturelement
- 7: Draht
- 8: Pulsmesser
- 9: LED
- 10: Atemtakter
- 11: Gehäuse
- 12: Signaleinheit
- 13: Brustband
- 14: Langloch
- 15: Bedienelement
- 16: Bedienelement

## Patentansprüche

1. Tragbarer Atemtakter
- mit einer Signaleinheit (5, 6) zum wahrnehmbaren Übertragen eines Signals auf den Körper des Benutzers und
- mit einer Steuereinheit zum periodischen Ansteuern der Signaleinheit (5, 6) mittels eines Ansteuersignals, wobei eine Periode des Ansteuersignals eine Einatmungsphase und eine Ausatmungsphase umfasst, wobei
das Ansteuersignal unabhängig von physiologischen Körperwerten gebildet ist, das Signal ein Vibrationssignal, ein Stromirnpulssignal und/oder ein Temperatursignal ist, und die Dauer der Einatmungsphase nicht länger als die Dauer der Ausatmungsphase ist, **dadurch gekennzeichnet, dass** mit mehreren Perioden eine voreingestellte Sequenz einer Atemübung gebildet ist und dass die zeitlichen Abstände der einzelnen Sequenzen untereinander einstellbar sind.

2. Tragbarer Atemtakter nach Anspruch 1, **dadurch gekennzeichnet, dass** die Periode eine Pausenphase umfasst.

3. Tragbarer Atemtakter nach Anspruch 1 oder 2, **gekennzeichnet durch** ein Befestigungsmittel (2) zum Befestigen des Atemtakters (1) am Körper oder an einem körpernahen Kleidungsstück eines Benutzers.

4. Tragbarer Atemtakter nach Anspruch 3, **dadurch gekennzeichnet, dass** das Befestigungsmittel (2) eine Ohrklammer, ein Ohreinsatz, ein Brustband, ein Armband, ein Fußband, eine Klettvorrichtung, ein Klebestreifen, eine Heftklemme, eine Heftklammer und/oder eine Öse ist.

5. Tragbarer Atemtakter nach einem der vorstehenden Ansprüche, **dadurch gekennzeichnet, dass** das Signal zusätzlich ein mechanisches, akustisches und/oder optisches Signal umfasst.

6. Tragbarer Atemtakter nach einem der vorstehenden Ansprüche, **dadurch gekennzeichnet, dass** die Periodendauer, die Dauer der Einatmungsphase, die Dauer der Ausatmungsphase und die Dauer der wenigstens einen optionalen Pausenphase einstellbar sind.

7. Tragbarer Atemtakter nach einem der vorstehenden Ansprüche, **dadurch gekennzeichnet, dass** die Einatmungsphase eine Dauer von 1 bis 10 Sekunden und die Ausatmungsphase eine Dauer von 2 bis 20 Sekunden aufweisen.

8. Tragbarer Atemtakter nach einem der vorstehenden Ansprüche, **dadurch gekennzeichnet, dass** die Signaleinheit (5, 6) einen Lautsprecher und/oder ein Vibrationselement (5) aufweist.

9. Tragbarer Atemtakter nach einem der vorstehenden Ansprüche, **dadurch gekennzeichnet, dass** das Signal während der Einatmungsphase eine Vibration zunehmender Intensität und/oder zunehmender Frequenz aufweist.

10. Tragbarer Atemtakter nach einem der vorstehenden Ansprüche, **dadurch gekennzeichnet, dass** das Signal während der Ausatmungsphase eine Vibration abnehmender Intensität und/oder abnehmender Frequenz aufweist.

11. Tragbarer Atemtakter nach einem der vorstehenden Ansprüche, **dadurch gekennzeichnet, dass** das Signal während der Einatmungsphase eine sich räumlich fortpflanzende oder ausbreitende Vibration und während der Ausatmungsphase eine sich entgegengesetzt zu der Einatmungsphase verhaltende Vibration aufweist.

12. Tragbarer Atemtakter nach einem der vorstehenden Ansprüche, **dadurch gekennzeichnet, dass** das Signal während der Einatmungsphase eine zunehmende Temperatur und während der Ausatmungsphase eine abnehmende Temperatur aufweist.

13. Tragbarer Atemtakter nach einem der vorstehenden Ansprüche, **dadurch gekennzeichnet, dass** die Steuereinheit eingerichtet ist, zu überwachen, ob eine vorgegebene Benutzungsdauer des tragbaren Atemtakters (1) in einer vorgegebenen Zeitspanne erreicht wird.

14. Tragbarer Atemtakter nach einem der vorstehenden Ansprüche, **dadurch gekennzeichnet, dass** der tragbare Atemtakter (1) wasserdicht ist.

## Claims

1. Portable breath timer
- with a signal unit (5, 6) for the perceptible transmission of a signal to the body of the user, and
- with a control unit for periodically actuating the signal unit (5, 6) by means of an actuation signal, wherein a period of the actuation signal comprises an inhalation phase and an exhalation phase, wherein
the actuation signal is formed independently of physiological values of the body, the signal is a vibration signal, a current pulse signal and/or a temperature signal, and the duration of the inhalation phase is not longer than the duration of the exhalation phase, **characterized in that** a preset sequence of a breathing exercise is formed by a number of periods and that the time intervals between the individual sequences can be set.

2. Portable breath timer according to Claim 1, **characterized in that** the period comprises a pause phase.

3. Portable breath timer according to Claim 1 or 2, **characterized by** a fastening means (2) for fastening the breath timer (1) to the body or to a piece of clothing of a user that is near the body.

4. Portable breath timer according to Claim 3, **characterized in that** the fastening means (2) is an ear clip an ear inset, a chest strap a wrist or arm band a foot band a Velcro device, an adhesive strip an attachment clasp an attachment clip and/or an eyelet.

5. Portable b eath time according to one of the preceding claims **characterized in that** the signal additionally comprises a mechanical acoustic and/or optical signal.

6. Portable breath timer according to one of the preceding claims, **characterized in that** the duration of the period the duration of the inhalation phase the duration of the exhalation phase and the duration of the at least one optional pause phase can be set.

7. Portable breath timer according to one of the preceding claims **characterized in that** the inhalation phase has a duration of 1 to 10 seconds and the exhalation phase has a duration of 2 to 20 seconds.

8. Portable breath timer according to one of the preceding claims, **characterized in that** the signal unit (5, 6) has a loudspeaker and/or a vibration element (5)

9. Portable breath time according to one of the preceding claims **characterized in that** during the inhalation phase the signal has a vibration of increasing intensity and/or increasing frequency.

10. Portable breath time according to one of the preceding claims **characterized in that** during the exhalation phase the signal has a vibration of decreasing intensity and/or decreasing frequency.

11. Portable breath timer according to one of the preceding claims, **characterized in that**, during the inhalation phase, the signal has a spatially propagating or spreading vibration and, during the exhalation phase, the signal has a vibration behaving oppositely from the inhalation phase.

12. Portable breath timer according to one of the preceding claims, **characterized in that** the signal has an increasing temperature during the inhalation phase and a decreasing temperature during the exhalation phase

13. Portable breath timer according to one of the preceding claims, **characterized in that** the control unit is designed to monitor whether a preset period of use of the portable breath timer (1) is reached in a preset period of time.

14. Portable breath timer according to one of the preceding claims, **characterized in that** the portable breath timer (1) is waterproof.

## Revendications

1. Cadenceur respiratoire portatif
- comprenant une unité de signalisation (5, 6) pour la transmission perceptible d'un signal vers le corps de l'utilisateur et
- comprenant une unité de commande pour piloter périodiquement l'unité de signalisation (5, 6) au moyen dun signal de pilotage, de sorte qu'une période du signal de pilotage inclut une phase d'inspiration et une phase d'expiration, dans lequel
le signal de pilotage est formé indépendamment de valeurs corporelles physiologiques, le signal est un signal de vibrations, un signal d'impulsion de courant et/ou un signal de température, et la durée de la phase d'inspiration n'est pas plus longue que la durée de la phase d'expiration,
**caractérisé en ce qu'**avec plusieurs périodes il se forme une séquence préétablie d'un exercice respiratoire, et **en ce que** les écarts temporels des séquences individuelles sont réglables les uns par rapport aux autres.

2. Cadenceur respiratoire portatif selon la revendication 1, **caractérisé en ce que** la période inclut une phase de pause

3. Cadenceur respiratoire portatif selon la revendication 1 ou 2, **caractérisé par** un moyen de fixation (2) pour la fixation du cadenceur respiratoire (1) sur le corps ou sur un élément d'habillage proche du corps d'un utilisateur

4. Cadenceur respiratoire portatif selon la revendication 3, **caractérisé en ce que** le moyen de fixation (2) est une pince à oreille, un insert d'oreille, une bande thoracique, un bracelet, un bracelet de pied, un dispositif agrippant, un ruban collant, une pmce d'agrafage, une agrafe et/ou un oeillet.

5. Cadenceur respiratoire portatif selon l'une des revendications précédentes, **caractérisé en ce que** le signal inclut en supplément un signal mécanique, acoustique et/ou optique.

6. Cadenceur respiratoire portatif selon l'une des revendications précédentes, **caractérisé en ce que** la durée de la période, la durée de la phase d'inspiration, la durée de la phase d'expiration et la durée de ladite au moins une phase de pause en option sont réglables.

7. Cadenceur respiratoire portatif selon l'une des revendications précédentes, **caractérisé en ce que** la phase d'inspiration présente une durée de 1 à 10 secondes et la phase d'expiration présente une durée de 2 à 20 secondes.

8. Cadenceur respiratoire portatif selon l'une des revendications précédentes, **caractérisé en ce que** l'unité de signalisation (5, 6) comprend un haut-parleur et/ou un élément à vibrations (5).

9. Cadenceur respiratoire portatif selon l'une des revendications précédentes, **caractérisé en ce que** pendant la phase d'inspiration le signal inclut une vibration d'intensité croissante et/ou de fréquence croissante.

10. Cadenceur respiratoire portatif selon l'une des revendications précédentes, **caractérisé en ce que** pendant la phase d'expiration le signal inclut une vibration d'intensité décroissante et/ou de fréquence décroissante.

11. Cadenceur respiratoire portatif selon l'une des revendications précédentes, **caractérisé en ce que** pendant la phase d'inspiration le signal inclut une vibration qui se perpétue ou qui se propage dans l'espace et **en ce que** pendant la phase d'expiration le signal inclut une vibration qui se comporte de façon opposée à la phase d'inspiration.

12. Cadenceur respiratoire portatif selon l'une des revendications précédentes, **caractérisé en ce que** pendant la phase d'inspiration le signal présente une température croissante et pendant la phase d'expiration le signal présente une température décroissante.

13. Cadenceur respiratoire portatif selon l'une des revendications précédentes, **caractérisé en ce que** l'unité de commande est conçue pour surveiller si une durée d'utilisation prédéterminée du cadenceur respiratoire portatif (1) est atteinte dans une période temporelle prédéterminée.

14. Cadenceur respiratoire portatif selon l'une des revendications précédentes, **caractérisé en ce que** le cadenceur respiratoire portatif (1) est étanche à l'eau.
